# EUROPEAN PATENT APPLICATION

(11) **EP 4 112 075 A1**
(43) Date of publication of application: **04.01.2023**
(21) Application number: 21759992.7
(22) Date of filing: 25.02.2021
(51) Int. Cl.: A61K 39/145, A61P 31/16, A61P 37/04

(54) **METHOD FOR PRODUCING INACTIVATED INFLUENZA VACCINE AND VACCINE COMPOSITION THEREOF**

(30) Priority: 26.02.2020 JP 2020030139
(71) Applicant: Denka Company Limited, Tokyo 103-8338 (JP)
(72) Inventor: MITSUMATA, Ryotaro, Gosen-shi, Niigata 959-1836 (JP); YOSHIDA, Keiko, Gosen-shi, Niigata 959-1836 (JP)
(74) Representative: Schiener, Jens
(86) International application number: PCT/JP2021/007073
(87) International publication number: WO 2021/172418

(57) **Abstract**

Provided is an inactivated influenza vaccine with high immunogenicity and a method for producing the same. The method for producing an inactivated influenza vaccine by performing inactivation treatment using formaldehyde comprises a step of treating a virus solution containing an influenza virus collected from a host with β-propiolactone in advance.

## Description

### Technical Field

The present invention relates to an inactivated influenza vaccine with high immunogenicity and a method for producing the same.

### Background Art

Human influenza viruses are single-stranded RNA viruses of the family Orthomyxoviridae and are classified into type A, type B, and type C according to the antigenicity of the internal antigen. Among them, viruses that cause a big epidemic every year mainly in winter are the type A and type B viruses, and also in Japan, estimated about 10 million people or more are known to be affected annually. The most effective means for preventing the influenza viruses that cause such a big epidemic every year is a vaccine. Vaccines against influenza viruses in Japan were introduced in the wake of the Asian flu epidemic in 1957, and the vaccines at the time were inactivated whole-virus vaccines obtained by inactivation treatment of purified influenza viruses.

Then, split vaccines obtained by disrupting virus particles by treatment with diethyl ether or a surfactant and removing lipid components for the purpose of reducing side reactions, such as fever, have been approved and are currently widespread globally. In contrast, in the vaccine against A/H5N1 subtype, which is a pre-pandemic vaccine, inactivated whole-virus particles are used as the antigen even today because of no history of infection or history of vaccination.

Inactivated whole-virus vaccines and split vaccines are both so-called inactivated vaccines in which the infectivity of the pathogen virus has disappeared. Examples of the inactivation treatment of a vaccine include treatment with β-propiolactone, formaldehyde, or diethyl ether or a surfactant to be used for disruption of virus particles (Non Patent Literature 1), and the most proven treatment used is formaldehyde treatment. The above-described inactivated whole virus vaccine and split vaccine are both vaccines inactivated with formaldehyde, and formaldehyde is used not only as an inactivating agent but also as a storage stabilizer, in the process for vaccine production or for one ingredient of a vaccine preparation.

In the meantime, inactivated whole-virus vaccines have higher primary immune effect (priming effect) compared with split vaccines. This effect is caused by incorporation of viral nucleic acid into cells to activate innate immunity via Toll-like receptor 7 (TLR7) (Akira S., Phil. Trans. R. Soc. B, 2011, 366: 2748-2755). It is said that the high priming effect by an inactivated whole-virus vaccine can be attributed to the high content of viral nucleic acid in the inactivated whole-virus vaccine. Although the content is low compared with that in inactivated whole-virus vaccines, split vaccines contain viral nucleic acids, which contributes not a little to the immunogenicity thereof.

Since formaldehyde is known to form crosslinking through a reaction with a primary amine of protein, the phenyl group of tyrosine and the like, and a primary amine of a nucleic acid base, it is concerned that excessive denaturation of viral nucleic acid by formaldehyde brings a reduction in immunogenicity in both inactivated whole-virus vaccines and split vaccines. Consequently, inactivation treatment with formaldehyde is required to be performed under appropriate conditions that do not decrease the innate immune activity.

### Citation List

### Non Patent Literature

[Non Patent Literature 1] Pawar SD, Murtadak VB, Kale SD, Shinde PV, and Parkhi SS, Evaluation of different inactivation methods for high and low pathogenic avian influenza viruses in egg-fluids for antigen preparation, J. Virol. Methods, 2015, Sep. 15, 222: 28-33

### Summary of Invention

### Technical Problem

The present invention relates to provision of an inactivated influenza vaccine with high immunogenicity and a method for producing the vaccine.

### Solution to Problem

The present inventors have studied diligently to prevent reduction in innate immune activation by a vaccine antigen when an influenza virus is inactivated with formaldehyde and, as a result, found surprisingly that a reduction in the innate immune activation capacity of an influenza vaccine antigen by formaldehyde can be prevented by treating the influenza virus in advance with β-propiolactone, which is used as a virus-inactivating agent like formaldehyde, and then treating the virus with formaldehyde.

That is, the present invention relates to the following items 1) to 5):
1) a method for producing an inactivated influenza vaccine by performing inactivation treatment using formaldehyde, the method including a step of treating a virus solution containing an influenza virus collected from a host with β-propiolactone in advance;
2) the method of 1), wherein the inactivation treatment using formaldehyde is performed by addition of formalin to the virus solution at a final concentration of 0.005 to 0.015 vol%;
3) the method of 2), wherein the inactivation treatment using formaldehyde is performed by a reaction at 2°C to 8°C for 3 to 14 days or at 20°C to 30°C for 3 days;
4) the method of any one of 1) to 3), wherein the β-propiolactone treatment is performed by addition of β-propiolactone to the virus solution containing the collected influenza virus at a final concentration of 0.0125 to 0.1 vol% and a reaction at 2°C to 8°C for 18 hours or longer; and
5) an inactivated whole-virus vaccine or split vaccine of an influenza virus, produced by the method according to any one of 1) to 4).

### Advantageous Effects of Invention

According to the method of the present invention, reduction in innate immune activation capacity of an influenza vaccine antigen due to formaldehyde treatment can be prevented while maintaining the pyrogenic activity-decreasing effect due to the formaldehyde treatment. Accordingly, the present invention can provide an inactivated influenza vaccine having high immunogenicity and possibly decreased pyrogenic activity and thus can highly contribute to the pharmaceutical industry.

### Brief Description of Drawings

[Figure 1] Figure 1 shows evaluation of the TLR7 activation capacity of influenza virus particles.
[Figure 2] Figure 2 shows a reduction in TLR activation capacity by formaldehyde treatment.
[Figure 3] Figure 3 shows resistance to formaldehyde by β-propiolactone treatment.
[Figure 4] Figure 4 shows (A): conditions for formaldehyde treatment and changes of TLR activity in B/Yamagata lineage antigen (reaction temperature: 4°C), and (B): conditions for formaldehyde treatment and changes of TLR activity in B/Yamagata lineage antigen (reaction temperature: 25°C).
[Figure 5] Figure 5 shows (A): conditions for formaldehyde treatment and changes of TLR activity in A/H3N2 subtype antigen (reaction temperature: 4°C), and (B): condition for formaldehyde treatment and changes of TLR activity in A/H3N2 subtype antigen (reaction temperature: 25°C) .
[Figure 6A] Figure 6A shows evaluation of the density increase of nucleoprotein by formaldehyde treatment (no treatment).
[Figure 6B] Figure 6B shows evaluation of the density increase of nucleoprotein by formaldehyde treatment (4°C, 0.02%, 3 days).
[Figure 6C] Figure 6C shows evaluation of the density increase of nucleoprotein by formaldehyde treatment (4°C, 0.02%, 14 days).
[Figure 6D] Figure 6D shows evaluation of the density increase of nucleoprotein by formaldehyde treatment (25°C, 0.02%, 3 days).
[Figure 6E] Figure 6E shows evaluation of the density increase of nucleoprotein by formaldehyde treatment (25°C, 0.02%, 14 days).

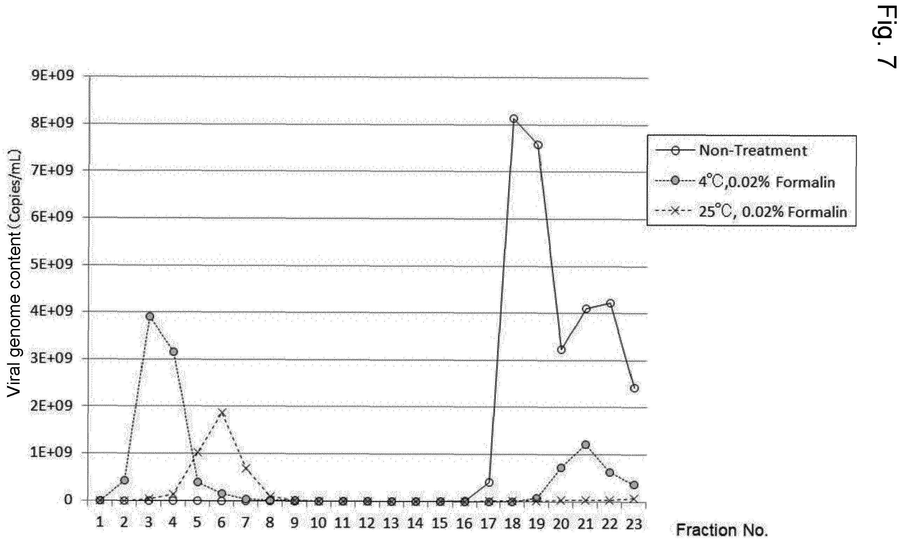

[Figure 7] Figure 7 shows evaluation of the density increase of viral genome by formaldehyde treatment.
[Figure 8A] Figure 8A shows the results (HI antibody titer) of mouse immunogenicity test (A/H1N1).
[Figure 8B] Figure 8B shows the results (HI antibody titer) of mouse immunogenicity test (A/H3N2).
[Figure 8C] Figure 8C shows the results (HI antibody titer) of mouse immunogenicity test (B/Victoria).
[Figure 9A] Figure 9A shows the results (antigen specific IgG titer) of mouse immunogenicity test (A/H1N1).
[Figure 9B] Figure 9B shows the results (antigen specific IgG titer) of mouse immunogenicity test (A/H3N2).
[Figure 9C] Figure 9C shows the results (antigen specific IgG titer) of mouse immunogenicity test (B/Victoria).
[Figure 10] Figure 10 shows pyrogenic activity evaluation in primates.

### Description of Embodiments

The following is a detailed description of suitable embodiments of the present invention. However, the present invention is not limited to the following embodiments.

In the present invention, the term "influenza virus" refers to influenza A virus or influenza B virus, or both thereof. The influenza virus encompasses all subtypes that are currently known and subtypes that will be isolated and identified in the future.

In the present invention, the term "influenza vaccine" means a vaccine containing at least one antigen of either influenza A virus or influenza B virus. That is, the influenza vaccine of the present invention may be a monovalent vaccine containing only one of influenza A virus and influenza B virus or may be a multivalent vaccine containing both of them. The antigen may be an inactivated whole-virus vaccine having no infectivity while maintaining the form of the virus particles or may be a split vaccine obtained by disrupting the virus particles with a surfactant or an organic solvent, such as diethyl ether.

The influenza virus strain that is used for preparing the vaccine of the present invention may be a strain isolated from an infected animal or patient or may be a recombinant virus established in culture cells by genetic engineering.

The method for producing the inactivated influenza vaccine of the present invention is a method of performing inactivation treatment using formaldehyde, wherein the method includes a step of treating a virus solution containing an influenza virus collected from a host with β-propiolactone in advance.

The virus solution to be treated with β-propiolactone is a virus solution containing an influenza virus obtained by infection to and culturing in a host and collection therefrom.

Here, the "host" used for proliferation of the influenza virus may be either a cultured cell or an embryonated chicken egg. That is, in the method of the present invention, the influenza virus as the target of treatment may be a virus proliferated by either an embryonated chicken egg method or a cell cultivation method.

The "embryonated chicken egg method" is a method for obtaining a virus solution containing virus particles by inoculating and culturing a virus strain in an embryonated chicken egg and then subjecting the virus suspension to clarification, concentration, purification, and inactivation.

Here, culturing is performed by inoculating the influenza virus into an embryonated chicken egg and culturing the virus at 30°C to 37°C for about 1 to 7 days, preferably at 33°C to 35°C for about 2 days. After the completion of the culturing, the virus suspension (infected allantoic fluid) is collected, and centrifugation or filtration is performed for clarification. Subsequently, ultrafiltration is performed for concentration. Viral purification can be performed by a means including ultracentrifugation such as sucrose density gradient centrifugation, or liquid chromatography.

The "cell cultivation method" is a method for obtaining a virus solution containing virus particles by inoculating and culturing a virus strain in cultured cells and performing clarification, concentration, purification, and inactivation as in the embryonated chicken egg method.

Here, the cultured cells are not particularly limited as long as the influenza virus is proliferative therein, and examples thereof include MDCK (Madin-Darby Canine Kidney), Vero, Caco-2, PER.C6, EB66, and these cells that have been recombined so as to express a high level of a receptor to be used for entry of a virus.

Culturing is performed by the embryonated chicken egg method or the cell cultivation method, and β-propiolactone treatment is performed at any period of the clarification, concentration, or purification of the collected influenza virus suspension.

In general, inactivation treatment by formaldehyde is performed at any period of the clarification, concentration, or purification of an influenza virus suspension. However, in the present invention, β-propiolactone treatment is performed in advance prior to the formaldehyde treatment.

That is, in the method of the present invention, the β-propiolactone treatment and the subsequent formaldehyde treatment can be performed before or after the clarification step, concentration step, and purification step, preferably before or after the purification step, and more preferably after the purification step. In the case of a split vaccine, the treatments may be performed before or after the step of disrupting a virus.

β-Propiolactone is a monoalkylating agent that is widely used for virus inactivation in preparation of many vaccines. In the present invention, the β-propiolactone treatment is, for example, a method by adding β-propiolactone to a virus solution containing the influenza virus collected after the culturing at a final concentration of 0.0125 to 0.1 vol%, preferably 0.025 to 0.075 vol%, and more preferably 0.05 vol% and performing a reaction at 2°C to 8°C for 18 hours or longer, preferably 20 hours or longer, and more preferably 24 hours or longer and 50 hours or shorter and preferably 30 hours or shorter.

As a more preferred aspect, for example, treatment with 0.05% at 2°C to 8°C for 24 hours is mentioned.

In the present invention, the formaldehyde treatment is performed for the virus solution treated with β-propiolactone, and the treatment condition is, for example, a step of adding formalin to the virus solution at a final concentration of 0.015 vol% or less, preferably 0.005 to 0.015 vol%, more preferably 0.01 to 0.015 vol%, and more preferably 0.01 vol% and performing a reaction at 2°C to 8°C for 14 days or less or at 20°C to 30°C for 3 days or less.

As a more preferred aspect, for example, it is mentioned to add formalin at a final concentration of 0.01 vol% and perform a reaction at 4°C for 3 to 14 days or at 25°C for 3 days.

In general, the formaldehyde treatment is performed at a final formalin concentration of 0.02 to 0.1 vol% at 2°C to 8°C for 14 days in order to exert the inactivation effect. In the present invention, the treatment can be performed under mild conditions as described above.

Incidentally, in the present invention, "formalin" means a formaldehyde aqueous solution containing 35% to 41% formaldehyde.

In the thus-produced influenza vaccine, the TLR activation capacity is not reduced, and the pyrogenic activity-decreasing effect by formalin treatment is maintained. Accordingly, the vaccine becomes an inactivated vaccine maintaining high immunogenicity and having possibly decreased pyrogenic activity.

Here, the phrase "TLR activation capacity is not reduced" means that the TLR stimulatory activity is not significantly reduced compared with an antigen treated with β-propiolactone alone or an influenza virus not subjected to inactivation treatment.

Incidentally, the TLR stimulatory activity can be measured by, for example, as described in Example described later, measuring a secretory alkaline phosphatase (SEAP) activity in the culture supernatant when the inactivated whole influenza antigen of the present invention is exposed to RAW264.7 cells into which a TLR7 gene and a secretory alkaline phosphatase (SEAP) gene have been incorporated.

In the inactivated influenza vaccine of the present invention, the amount as the hemagglutinin is 7.5 µg or more per strain of virus, i.e., 7.5 µg or more HA/strain, preferably 9 to 21 µg HA/strain, and more preferably 15 µg HA/strain. Incidentally, the hemagglutinin content is the value obtained by measurement by a test method defined by WHO or national standards, such as single radial immunodiffusion assay. The antigen amount contained in the vaccine may be appropriately changed according to the type of the virus or the administration target.

The inactivated influenza vaccine of the present invention may further include a pharmaceutically acceptable carrier, in addition to the influenza virus antigen. Examples of the carrier include carriers that are usually used in production of vaccines, specifically, the examples include a buffer, an emulsifier, a preservative, an isotonizing agent, a pH adjuster, and an adjuvant.

The formulation of the inactivated influenza vaccine of the present invention may be, for example, a liquid, a lyophilized powder, a capsule, or a tablet.

The administration route of the inactivated influenza vaccine of the present invention may be, for example, subcutaneous administration, intramuscular administration, intracutaneous administration, nasal administration, sublingual administration, or oral administration, and the administration method may be, for example, a method by a syringe, a microneedle, a syringe with a microneedle, a transdermal patch, or a spray.

Examples of the administration target of the inactivated influenza vaccine of the present invention include humans and mammals other than humans, and humans are preferable. Examples of the mammals other than humans include, a mouse, a rat, a guinea pig, a rabbit, a pig, a cow, a horse, a goat, sheep, a dog, a cat, a rhesus monkey, a cynomolgus monkey, an orangutan, and a chimpanzee.

### Examples

The present invention will be more specifically described by Examples, but the present invention is not limited thereto.

### Reference Example 1 Evaluation of TLR activation capacity of influenza virus

Viruses of the B/Phuket/3073/2013 strain were inoculated in the chorioallantoic cavity of a 12-day old embryonated chicken egg and cultured for 3 days, and the chorioallantoic fluid was collected. The collected chorioallantoic fluid was clarified by filter filtration, then adsorbed on barium sulfate and eluted with a 12% sodium citrate solution to collect the influenza virus. The collected virus was further purified by performing replacement of the solution with a 6.7 mM phosphate buffered physiological saline solution (pH 7.2) by ultrafiltration and, after the buffer replacement, sucrose density gradient centrifugation to collect a fraction containing the influenza virus. The resulting virus solution is referred to as a purified influenza virus solution. β-Propiolactone, which is an inactivating agent, was added to this purified influenza virus solution at a final concentration of 0.05% to inactivate the infectivity of the influenza virus by a reaction at 4°C for 24 hours. After this inactivation reaction, the buffer was replaced with a 6.7 mM phosphate buffered physiological saline solution (pH 7.2) containing 1 w/w% sucrose by ultrafiltration (MWCO: 100,000) to obtain a β-propiolactone-treated inactivated whole-virus vaccine.

To 1.5 × 10⁶ cells of RAW264.7 (NBP2-26261) into which a secretory alkaline phosphatase gene had been incorporated, ODN2088 (Miltenyi Biotec K.K.), which is an antagonist of TLR7/8/9, was added in a final concentration of 0, 0.1, 1 and 10 µM. To the cells to which ODN2088 was added, the inactivated whole-virus vaccine of B/Phuket/3073/2013 strain prepared as described above was added in a total protein amount of 5 µg or 5 µg of imiquimod was added, and the cells were cultured under the conditions of 37°C and 5% CO₂ for 24 hours. After the culturing, the supernatant was collected, the alkaline phosphatase activity of the supernatant was measured with SEAP Reporter Assay Kit (trade name, Cayman Chemical Company), and in each of the inactivated whole-virus vaccine and imiquimod, the relative value of the signal of ODN2088 at each concentration with respect to the signal when ODN2088 was not added was calculated as the relative activity value.

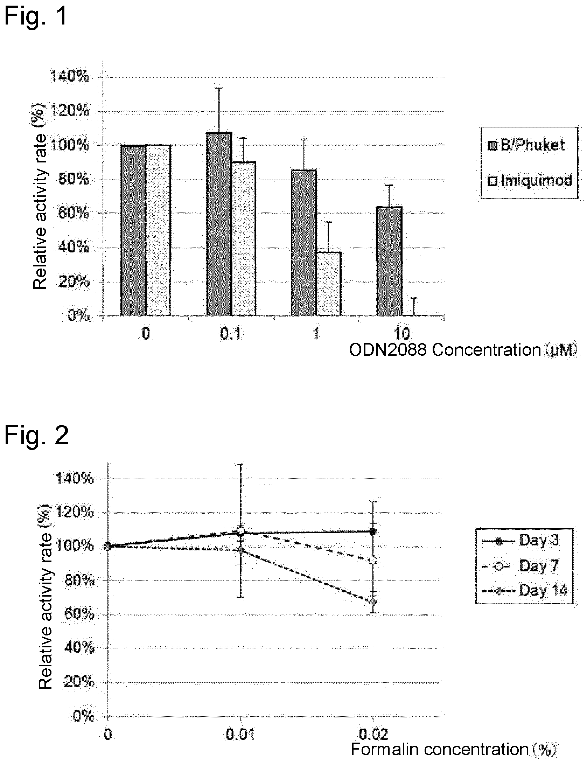

The relative activity values when pretreated with each concentration of ODN2088 are shown in Figure 1. It was confirmed that the relative activity values of the inactivated whole-virus vaccine and imiquimod tend to be decreased depending on the concentration of ODN2088. Consequently, it was considered that the TLR7 activation capacity possessed by the inactivated whole-virus vaccine can be evaluated by an in-vitro system using NBP2-26261.

### Reference Example 2 Decrease in TLR activation capacity by formaldehyde treatment

The purified influenza virus solution prepared by the method described in Reference Example 1 was adjusted such that the protein concentration was 200 µg/mL, and 10% formalin neutral buffer solution (FUJIFILM Wako Pure Chemical Corporation, formaldehyde content: 3.8% to 4.1%) was added thereto such that the final concentration of formalin was 0, 0.01%, or 0.02%, followed by a reaction at 4°C for 3, 7, or 14 days. After the reaction, glycine was added at a final concentration of 10 mM to stop the reaction of formaldehyde, and centrifugation was performed at 1,000,000×g at 4°C for 4 hours. The supernatant after the centrifugation was discarded, the resulting pellet of the virus was suspended in 6.7 mM phosphate buffered saline, and this suspension was used as a formaldehyde-treated virus.

The formaldehyde-treated viruses were each added to 1.5 × 10⁶ cells of NBP2-26261 in a total protein amount of 10 µg and were cultured under the conditions of 37°C and 5% CO₂ for 24 hours. After the culturing, the supernatant was collected, and the alkaline phosphatase activity of the supernatant was measured with SEAP Reporter Assay Kit (trade name, Cayman Chemical Company). From the measurement results, the relative value with respect to the signal when formalin was not added (formalin concentration: 0) was calculated for each sample, and the resulting value was used as the relative activity value.

As the results shown in Figure 2, it was confirmed that the relative activity value tends to be decreased with increases in the concentration and the number of days of the reaction in the formaldehyde treatment. Accordingly, it was considered that the TLR activation capacity is decreased by strong formaldehyde treatment to thereby cause reductions in the innate immune activity and the immunogenicity.

### Example 1 Resistance to formaldehyde acquired by β-propiolactone treatment

The purified influenza virus solution and β-propiolactone-treated inactivated whole-virus vaccine prepared in the above-described Reference Example 1 were each adjusted such that the protein concentration was 200 µg/mL, and 10% formalin neutral buffer solution (FUJIFILM Wako Pure Chemical Corporation, formaldehyde content: 3.8% to 4.1%) was added thereto such that the final concentration of formalin was 0, 0.01%, or 0.02%. After the addition of formalin, the reaction was performed at 4°C for 14 days. After the reaction, glycine was added at a final concentration of 10 mM to stop the reaction of formaldehyde, and centrifugation was performed at 1,000,000×g at 4°C for 4 hours. The supernatant after the centrifugation was discarded, and the resulting pellet of the virus was suspended in 6.7 mM phosphate buffered saline to prepare each sample.

To 1.5 × 10⁶ cells of NBP2-26261, the prepared samples were each added in a total protein amount of 10 µg and the cells were cultured under the conditions of 37°C and 5% CO₂ for 24 hours. After the culturing, the supernatant was collected, and the alkaline phosphatase activity of the supernatant was measured with SEAP Reporter Assay Kit (trade name, Cayman Chemical Company). From the measurement results, the relative value with respect to the signal when formalin was not added (formalin concentration: 0) was calculated for each sample, and the resulting value was used as the relative activity value.

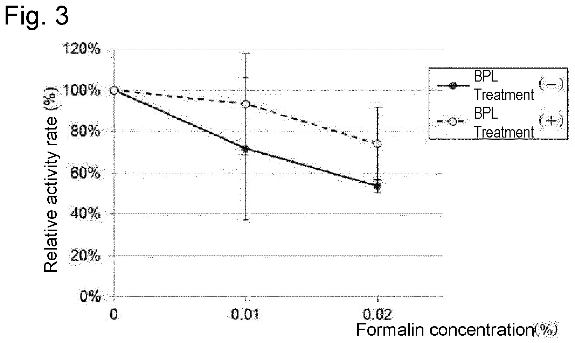

As shown in Figure 3, it was revealed that the reduction in TLR activation capacity by formaldehyde can be prevented by performing β-propiolactone treatment in advance. Accordingly, it was considered that an influenza vaccine antigen maintaining its innate immune activation capacity can be prepared by performing formaldehyde treatment before formaldehyde treatment.

### Example 2 Optimization of formaldehyde treatment condition

β-Propiolactone-treated inactivated whole-virus vaccines of B/Phuket/3073/2013 strain (B/Yamagata lineage) and A/Kansas/14/2017 strain (A/H3N2 subtype) were prepared by the same method as the method described in Reference Example 1, and 10% formalin neutral buffer solution (FUJIFILM Wako Pure Chemical Corporation, formaldehyde content: 3.8% to 4.1%) was added to the vaccine of each strain such that the final concentration of formalin was 0, 0.01%, or 0.02%. After the addition of formalin, the reaction was performed at 4°C or 25°C for 3, 7, or 14 days. After the reaction, glycine was added at a final concentration of 10 mM to stop the reaction of formaldehyde, and centrifugation was performed at 1,000,000×g at 4°C for 4 hours. The supernatant after the centrifugation was discarded, and the resulting pellet of the virus was suspended in 6.7 mM phosphate buffered saline to prepare each sample.

To 1.5 × 10⁶ cells of NBP2-26261, the prepared samples were each added in a total protein amount of 10 µg, and the cells were cultured under the conditions of 37°C and 5% CO₂ for 24 hours. After the culturing, the supernatants were each collected, and the alkaline phosphatase activity of the supernatant was measured with SEAP Reporter Assay Kit (trade name, Cayman Chemical Company). From the measurement results, the relative value with respect to the signal when formalin was not added (formalin concentration: 0) was calculated for each sample, and the resulting value was used as the relative activity value.

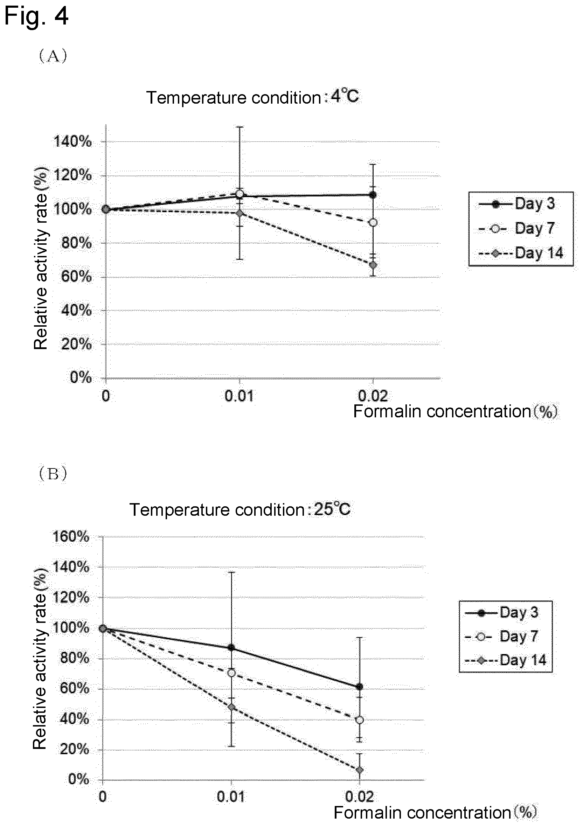

Figure 4 shows the influences of formaldehyde treatment on the β-propiolactone-treated inactivated whole-virus vaccine of the B/Phuket/3073/2013 strain (B/Yamagata lineage). In the reaction condition of 4°C, no clear reduction in the TLR activation capacity was observed in 3 to 14 reaction days at the formalin concentration of 0.01% (Figure 4(A)), and in the reaction condition of 25°C, a tendency of a reduction in the TLR activation capacity with an increase in the formalin concentration was observed regardless of the number of days of the reaction (Figure 4(B)). Table 1 below summaries the average values of relative activity values. Since this evaluation system performs quantitative evaluation using cells based on enzyme activity, a change of 30% or more was judged as a significant reduction in view of the variations in the evaluation method. Accordingly, it was considered that a significant reduction of the TLR activation capacity was observed in the conditions indicated by underlined values in the table.

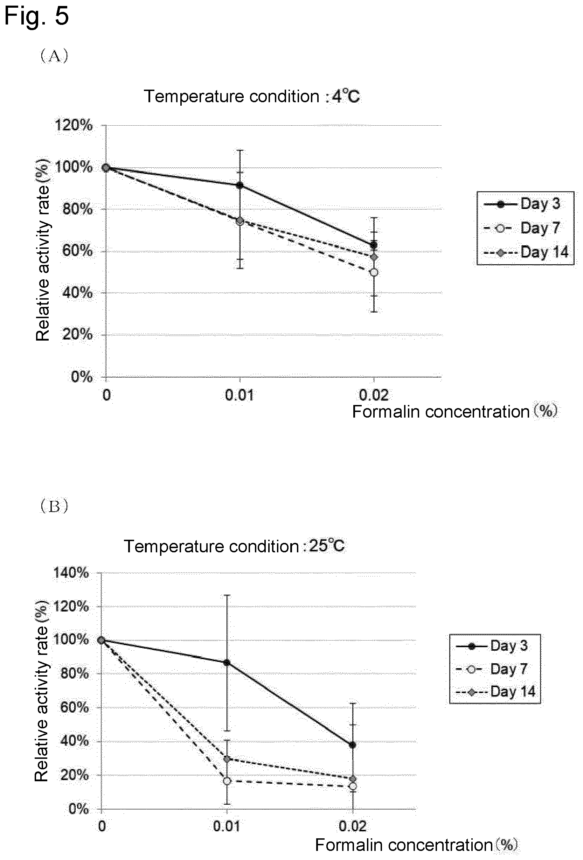

On the other hand, in the influences of formaldehyde treatment on the β-propiolactone-treated inactivated whole-virus vaccine of the A/Kansas/14/2017 strain (A/H3N2 subtype), as shown in Figure 5, a clear reduction in the TLR activation capacity was observed regardless of the number of days of the reaction at the formalin concentration of 0.02% even at 4°C (Figure 5(A)). In addition, at 25°C, a large reduction in the TLR activation capacity was observed in the reaction for 7 days or more even at the formalin concentration of 0.01% (Figure 5(B)). Table 2 below shows the relative activity values in the A/Kansas/14/2017 strain. Unlike the B/Phuket/3073/2013 strain, a change of 30% or less was observed in the reaction at 4°C with a formalin concentration of 0.01% for 3 to 14 days or the reaction at 25°C with a formalin concentration of 0.01% for 3 days, and in other reactions, a reduction by more than 30% in the TLR activation capacity was observed.

Accordingly, although inactivation treatment of viruses has conventionally been performed by a long-time reaction at a formalin concentration of 0.02% at 4°C, it is suggested that sensitivity to formaldehyde is high in some strains, and the TLR activation capacity is decreased. Accordingly, for both virus strains of type A and type B, the TLR activation capacity can be maintained by performing β-propiolactone treatment and then performing formaldehyde treatment with a low concentration of formalin, for example, at room temperature for several days. In the conventional formaldehyde treatment conditions, the treatment was performed at a relatively high concentration for a long time in order to achieve inactivation of viruses. However, in the present invention, sufficient inactivation can be achieved even in relatively mild formaldehyde treatment conditions by performing β-propiolactone treatment in advance.

**[Table 1]**

| Relative activity value in B/Phuket/3073/2013 strain | | | | | | |
|---|---|---|---|---|---|---|
| | 4°C | | | 25°C | | |
| Formalin concentration (%) | Day 3 | Day 7 | Day 14 | Day 3 | Day 7 | Day 14 |
| 0.01 | 107.9 | 109.4 | 98.0 | 87.2 | 70.4 | 48.0 |
| 0.02 | 108.6 | 92.3 | 67.3 | 61.1 | 39.9 | 6.4 |

**[Table 2]**

| Relative activity value in A/Kansas/14/2017 strain | | | | | | |
|---|---|---|---|---|---|---|
| | 4°C | | | 25°C | | |
| Formalin concentration (%) | Day 3 | Day 7 | Day 14 | Day 3 | Day 7 | Day 14 |
| 0.01 | 91.5 | 74.2 | 74.8 | 86.6 | 17.0 | 29.8 |
| 0.02 | 62.7 | 49.9 | 57.4 | 37.7 | 13.6 | 18.3 |

### Reference Example 3 Formation of crosslinking between viral genome and nucleoprotein (NP) by formaldehyde treatment

A β-propiolactone-treated inactivated whole-virus vaccines of B/Phuket/3073/2013 strain (B/Yamagata lineage) was prepared by the same method as the method described in Reference Example 1, and formaldehyde treatment was performed under the conditions shown in Table 3. After each reaction, glycine was added at a final concentration of 10 mM to stop the reaction of formaldehyde, and centrifugation was performed at 1,000,000×g at 4°C for 4 hours. The supernatant after the centrifugation was discarded, and the resulting pellet of the virus was suspended in 6.7 mM phosphate buffered saline. Sodium dodecyl sulfate was added to each suspension at a final concentration of 1%, followed by sucrose density gradient centrifugation (4°C, 206,500×g, 3 hours) with a fraction density of 10 to 60 w/w% to fractionate into 23 fractions.

Equal amounts of each fraction solution and SDS-PAGE sample buffer (8% SDS, 40% glycerol/250 mM Tris-HCl Buffer, pH 6.8) were mixed and reacted at 95°C for 5 minutes, followed by Western blotting. In addition, 12.5 µL of Proteinase K (F. Hoffmann-La Roche Ltd.) was added to 100 µL of each fraction solution of conditions 1, 3, and 5, and the reaction was performed at 70°C for 15 minutes, followed by qPCR for quantitative measurement of viral genome. The Western blotting was performed according to the method described below. In the qPCR, the viral genome was extracted with High Pure Viral RNA Kit (F. Hoffmann-La Roche Ltd.), and the genome of type B virus was quantitatively measured by the method described in Manual on Influenza diagnosis (4th edition) using the NS gene of type B as the target.

For the Western blotting, the fraction solution containing a sample buffer was subjected to electrophoresis on 12.5% polyacrylamide gel (ePAGEL, manufactured by Atto Corporation), and the reaction for transfer to a PVDF membrane was performed with a semi-dry transfer device (manufactured by Atto Corporation). The PVDF membrane after the transfer was immersed in 75 mL of a blocking buffer (TBS containing 10% skimmed milk) to perform a masking reaction at room temperature for 4 hours. After the reaction, the PVDF membrane was washed with an appropriate amount of TBS three times and was then immersed in an anti-NP antibody solution (LifeSpan BioSciences, Inc.) for a reaction at 4°C for about 16 hours (primary antibody response). After the primary antibody response, the PVDF membrane was washed with TBS containing Tween 20 (trade name) five times. An HRPlabeled anti-mouse antibody (Jackson Immuno Research Laboratories, Inc.) solution was added to the membrane, and a reaction was performed at room temperature for 60 minutes (secondary antibody response). After the secondary antibody response, the PVDF membrane was washed with TBS containing Tween 20 (trade name) five times, and nucleoprotein was detected with Super Signal (trade name) Western Lightning-Plus ECL (trade name, manufactured by PerkinElmer Co., Ltd.).

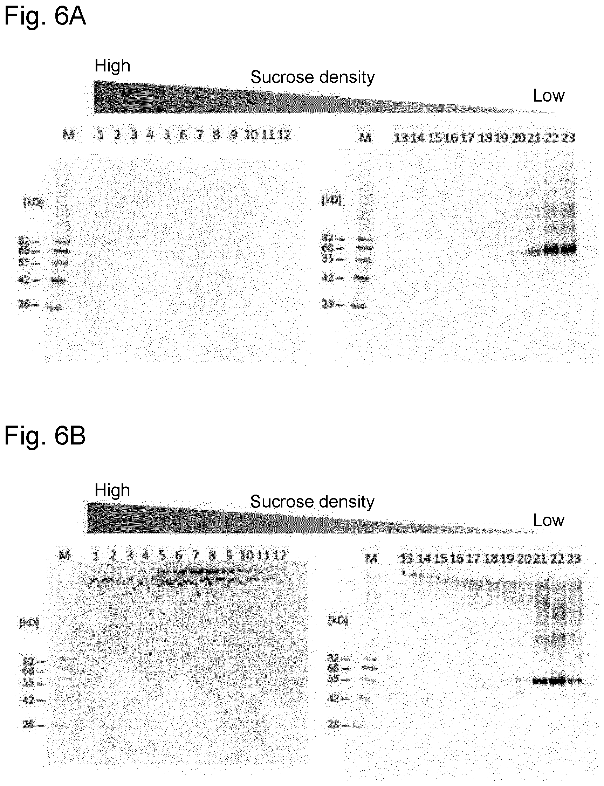

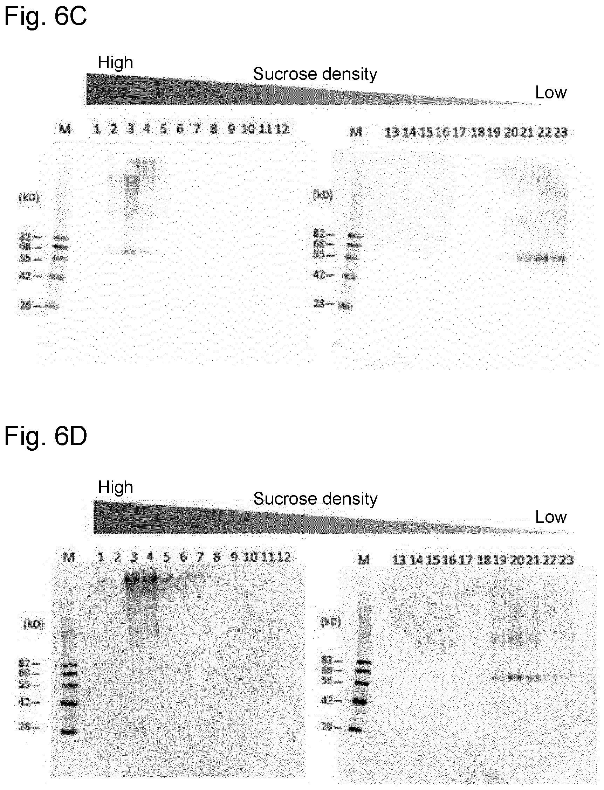

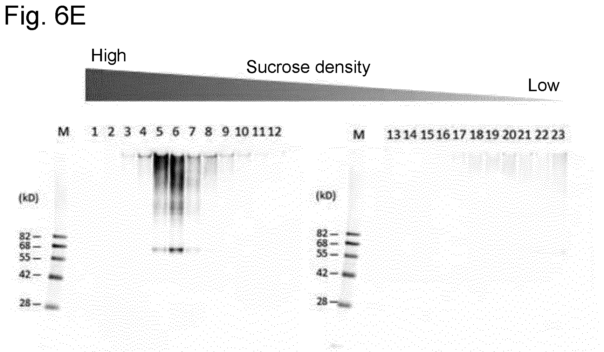

Figure 6 shows the results of Western blotting. In the reaction without formaldehyde treatment (Condition 1, Figure 6A) and reaction at a formalin concentration of 0.02% at 4°C for 3 days (Condition 2, Figure 6B), the nucleoprotein was detected only at the low density. In the reaction at a formalin concentration of 0.02% at 4°C for 14 days (Condition 3, Figure 6C) and reaction at a formalin concentration of 0.02% at 25°C for 3 days (Condition 4, Figure 6D), the nucleoprotein was detected at the high density and the low density. Furthermore, in the reaction at 0.02% at 25°C for 14 days (Condition 5, Figure 6E), the nucleoprotein was detected only at the high density. It was confirmed from these results that the nucleoprotein shifts to higher density by strengthening the formaldehyde treatment.

In the results of quantitative measurement of viral genome by qPCR performed under conditions 1, 3, and 5, it was revealed that as in the detection of nucleoprotein, the viral genome was detected only at the low density in condition 1 and was detected at the high density and the low density in condition 3 and that the viral genome was located only at the high density in condition 5. It is considered that decrease in the quantitative value of a viral genome with an increase in the strength of the formaldehyde treatment is caused by inhibition of the polymerase chain reaction attributable to the partially remaining crosslinking reaction of formaldehyde. The detected fraction of the viral genome is almost the same as the nucleoprotein fraction detected by Western blotting. Accordingly, it was considered that a nucleoprotein and a viral genome form crosslinking by formaldehyde treatment, and the TLR activation capacity is decreased when this crosslinking reaction excessively proceeds.

**[Table 3]**

| No | Formalin concentration (%) | Reaction temperature (°C) | Number of days of reaction (day) |
|---|---|---|---|
| Condition 1 | 0 | - | - |
| Condition 2 | 0.02 | 4 | 3 |
| Condition 3 | 0.02 | 4 | 14 |
| Condition 4 | 0.02 | 25 | 3 |
| Condition 5 | 0.02 | 25 | 14 |

### Example 3 Mouse immunogenicity test

As in the method described in Example 2, the following antigens for three strains (A/H1N1: A/Brisbane/02/2018, A/H3N2: A/Kansas/14/2017, and B/Victoria lineage: B/Maryland/15/2016) were prepared: 1) a β-propiolactone-treated inactivated whole particle antigen, 2) an inactivated whole particle antigen treated with formalin at a final concentration of 0.01% at 4°C for 14 days after β-propiolactone treatment, and 3) an inactivated whole particle antigen treated with formalin at a final concentration of 0.02% at 25°C for 14 days after β-propiolactone treatment. Each antigen was adjusted such that the hemagglutinin of each strain was 15 µg HA per 0.2 mL with a 6.7 mM phosphate buffered physiological saline solution (pH 7.2) containing 1 w/w% sucrose and was subjected to a mouse immunogenicity test as an inactivated whole-virus vaccine (WV).

The inactivated whole-virus vaccine and an influenza HA vaccine (split vaccine, SV) as a control were administered subcutaneously on the back of 5-week old female BALB/c mice in an amount of 15 µg HA/administration. On the 21st day after the administration, the HI antibody titer and the antigen specific IgG titer of the serum obtained from each mouse were measured.

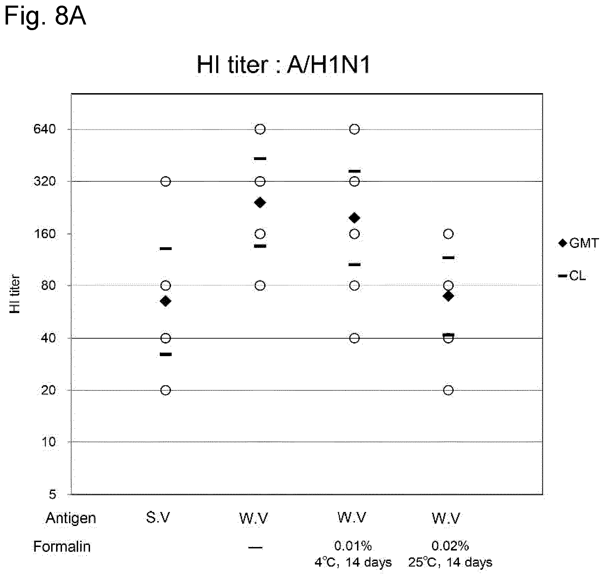

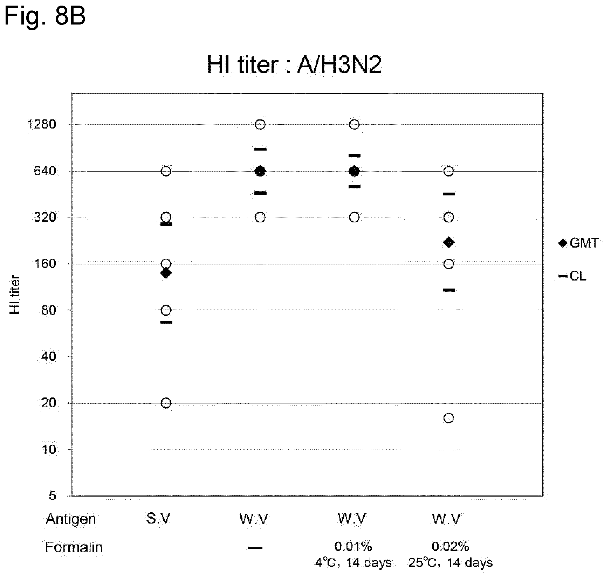

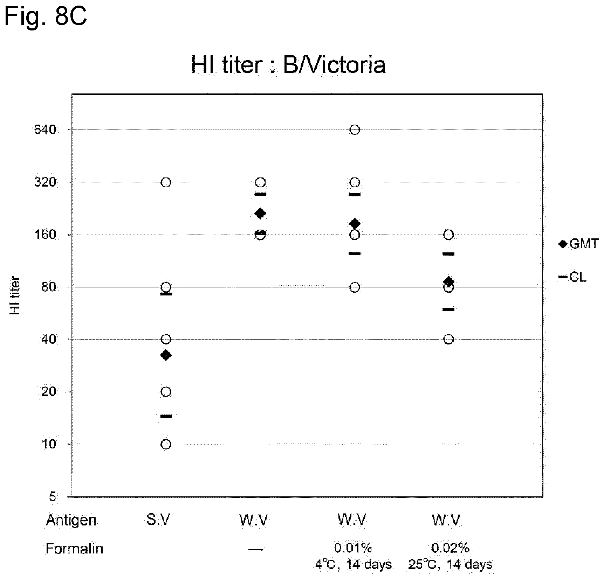

The results of HI antibody titer measurement are shown in Figures 8A to 8C. High antibody induction was observed in WV subjected to treatment with β-propiolactone alone compared with SV for each strain, and the antibody induction in "WV treated with 0.01% formalin at 4°C for 14 days (WV-0.01)" without showing a decrease in the innate immune activation capacity was almost equal to that in WV subjected to β-propiolactone treatment only. In "WV treated with 0.02% formalin at 25°C for 14 days (WV-0.02)" showing a large decrease in the innate immune activation capacity, it was confirmed that in all strains, the antibody induction was significantly decreased compared with WV-0.01 (Mann-Whitney test, p < 0.05).

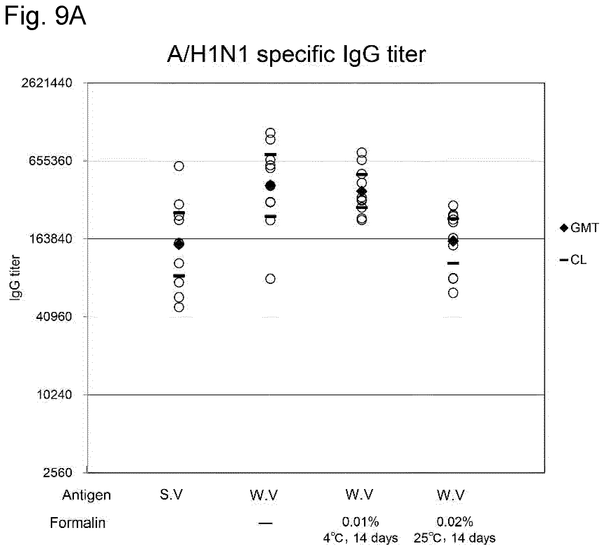

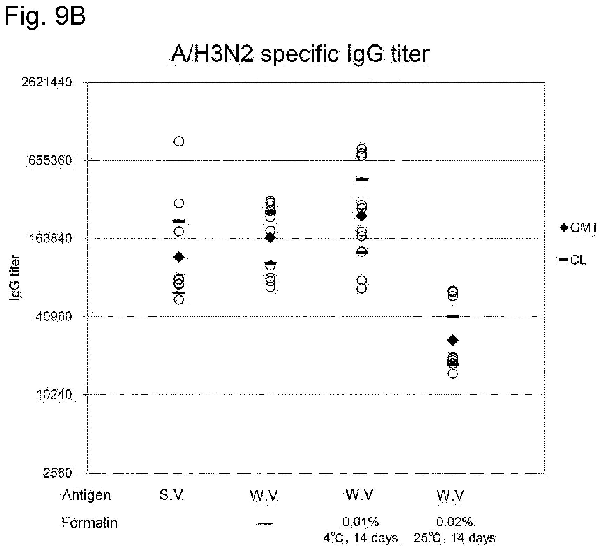

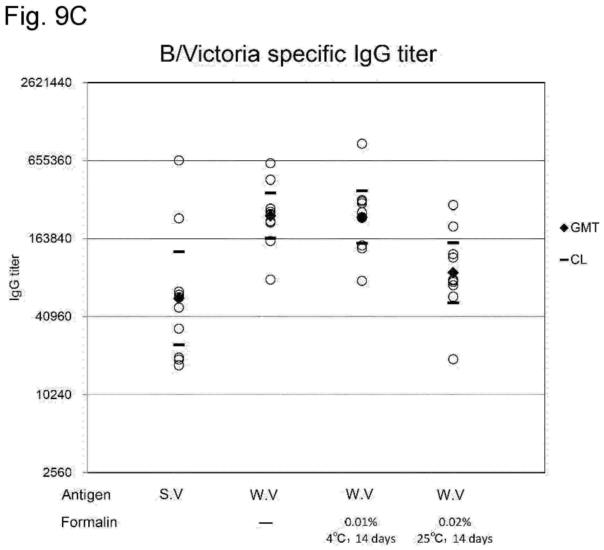

The results of the antigen specific IgG titer are shown in Figures 9A to 9C. The same tendency as in the HI antibody titer was observed. The antibody induction in WV-0.01 was almost equal to that in WV subjected to β-propiolactone treatment alone, and it was confirmed that the antibody induction in WV-0.02 was significantly decreased compared with WV-0.01 (Mann-Whitney test, p < 0.01) .

Accordingly, it was shown that the innate immune activation capacity and the antibody induction capacity correlate to each other, and in order to maintain a high immunogenicity of an influenza vaccine, it is important to maintain the original activity without losing the innate immune activation capacity possessed by the vaccine antigen.

### Example 4 Pyrogenic activity evaluation in primates

Atropine sulfate hydrate was intramuscularly administered as preanesthetic medication to 12 quarantined cynomolgus monkeys, and then ketamine hydrochloride was intramuscularly administered to sedate the animals. After the sedation, a telemetry transmitter (TL11M2-D70-PCT, Data Sciences International Inc.) was implanted in the abdominal cavity and fixed to the abdominal wall under isoflurane inhalation anesthesia. Immediately before the surgery, an antibiotic for prevention of infection and ketoprofen for pain alleviation were intramuscularly administered. An observation period of about 3 weeks was set after the implant surgery of the telemetry transmitter, and animals were tested after being verified that there were no abnormalities.

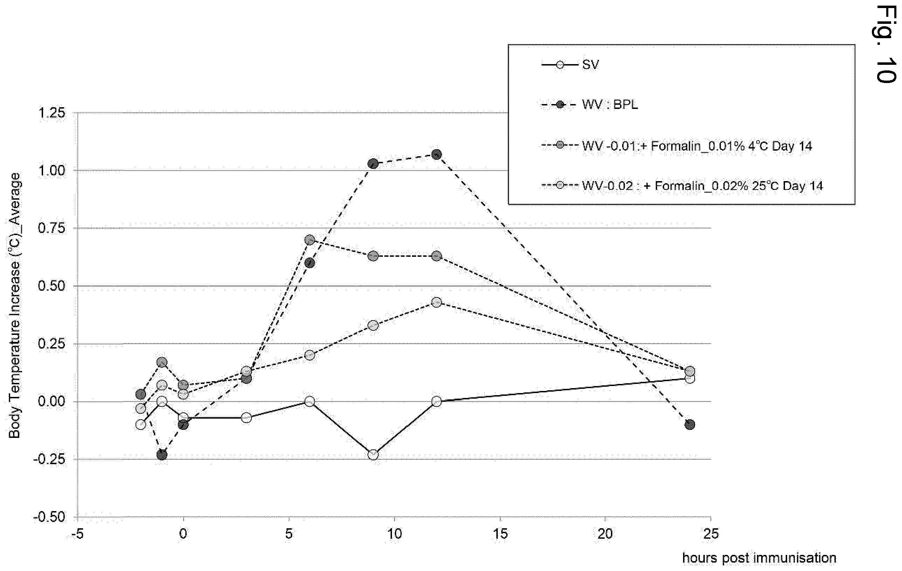

To the individual cynomolgus monkeys implanted with the telemetry transmitter, 0.5 mL of a physiological saline solution (Otsuka Pharmaceutical Factory, Inc.) was administered (Day 0), and the body temperature 24 hours after the administration was measured (measurement of baseline of each individual). On the 7th day after the administration of the physiological saline solution, 0.5 mL of any of various tetravalent inactivated whole-virus vaccines (15 µg HA/strain/0.5 mL, WV) and an influenza HA vaccine (15 µg HA/strain/0.5 mL, SV) prepared as in Example 3 was administered to each individual. The body temperature 24 hours after the administration was measured, and the difference from the baseline measurement value was calculated for each individual. The differences in the body temperature were defined as fever, and the results are summarized in Figure 10.

As shown in Figure 10, pyrogenic activity was not observed in the influenza HA vaccine, which is a split vaccine, and the highest pyrogenic activity was observed in the inactivated whole-virus vaccine subjected to treatment with β-propiolactone alone. It was revealed that, in contrast to this inactivated whole-virus vaccine subjected to treatment with β-propiolactone alone, all the inactivated whole-virus vaccine subjected to formalin treatment had decreased pyrogenic activity. In comparison between WV-0.01 and WV-0.02, although the pyrogenic activity of WV-0.02 having decreased innate immune activation capacity was lower than the other, the difference in the peak values of fever was 0.4°C (WV-0.01: 0.7°C, WV-0.02: 0.3°C).

Accordingly, the pyrogenic activity is decreased by formalin treatment, and the decrease in pyrogenic activity is larger when the treatment is performed under stronger (high concentration, high temperature, and long period) conditions. However, since WV-0.02 shows a significant decrease in immunogenicity as shown in Example 3, a vaccine excellent in both effectiveness and safety is a vaccine having possibly reduced pyrogenic activity without losing the innate immune activation capacity like WV-0.01.

## Claims

1. A method for producing an inactivated influenza vaccine by performing inactivation treatment using formaldehyde, the method comprising a step of treating a virus solution containing an influenza virus collected from a host with β-propiolactone in advance.

2. The method according to claim 1, wherein the inactivation treatment using formaldehyde is performed by addition of formalin to the virus solution at a final concentration of 0.005 to 0.015 vol%.

3. The method according to claim 2, wherein the inactivation treatment using formaldehyde is performed by a reaction at 2°C to 8°C for 3 to 14 days or at 20°C to 30°C for 3 days.

4. The method according to any one of claims 1 to 3, wherein the β-propiolactone treatment is performed by addition of β-propiolactone to the virus solution containing the collected influenza virus at a final concentration of 0.0125 to 0.1 vol% and a reaction at 2°C to 8°C for 18 hours or longer.

5. An inactivated whole-virus vaccine or split vaccine of an influenza virus, produced by the method according to any one of claims 1 to 4.
